# EUROPEAN PATENT APPLICATION

(11) **EP 4 403 140 A1**
(43) Date of publication of application: **24.07.2024**
(21) Application number: 23152646.8
(22) Date of filing: 20.01.2023
(51) Int. Cl.: A61F 2/30

(54) **AUGMENTATION COMPONENT FOR AN ARTICULAR OR JOINT BONE IMPLANT**

(71) Applicant: Aesculap AG, 78532 Tuttlingen (DE)
(72) Inventor: Lang, Rachel, 52000 Chaumont (FR); Moussa, Said, 52000 Chamarandes-Choignes (FR); Ozkan, Rasit, 52000 Chamarandes-Choignes (FR); Zouaghi, Housseyn, 52000 Chaumont (FR)
(74) Representative: Patentanwälte Ruff, Wilhelm, Beier, Dauster & Partner mbB

(57) **Abstract**

The invention refers to an augmentation component (1) for an articular or joint implant (30), wherein the augmentation component (1) comprises
- a first structure (12) being adapted to allow passage or penetration of a biocompatible material (20) being curable on contact with water or an aqueous liquid and/or being adapted to allow in-growth of bone tissue and
- a second structure (15) surrounding the first structure (12) at least in sections or being arranged at least in sections onto the first structure (12) along a periphery of the first structure (12).

Further, the invention refers to a surgical kit or system (10) comprising, in particular spatially separated from each other, the above augmentation component (1) and at least one further kit or system component.

## Description

### FIELD OF THE INVENTION

The present invention relates to an augmentation component for an articular or joint bone implant and a surgical kit or system for treating an articular or joint bone defect.

### BACKGROUND OF THE INVENTION

During arthroplasty (e.g. knee arthroplasty) it is possible for the patient to suffer from a significant bone loss. Therefore, augments may be used to adapt the treatment to the patient. The augments are used to compensate for a possible bone loss.

In WO 2008/040408 A1 is described an augmentation component for bone implants. The augmentation component is described having a metallic porous structure and at least one opening for a securing element for securing the augmentation component to the bone implant. The augmentation component aims at ensuring the stability of the bone implant and allowing for optimum in-growth of bone tissue.

Despite the above approach there is still further need of further improvement, in particular since the augmentation component known from WO 2008/040408 A1 is intended for use only without bone cement.

### Object and solution

In view of the foregoing, the object underlying the present invention is therefore to make available an augmentation component and a surgical kit which properly address the above-mentioned need.

This object is properly accomplished by an augmentation component according to claim 1 and by a surgical kit according to claim 15. Preferred embodiments of the invention are defined in the dependent claims and in the present description. The subject-matter and wording, respectively, of all claims is hereby incorporated into the description by explicit reference.

According to a first aspect, the present invention refers to an augmentation component, in particular for an articular or joint implant.

The augmentation component comprises
- a first structure being adapted to allow passage or penetration of a biocompatible material being curable on contact with water or an aqueous liquid and/or being adapted to allow in-growth of bone tissue and
- a second structure surrounding the first structure at least in sections, in particular only in sections or continuously, i.e. completely or without any interruptions, or being arranged at least in sections, in particular only in sections or continuously, i.e. completely or without any interruptions, onto the first structure along a periphery of the first structure.

The term "augmentation component" as used according to the present invention refers to a component which is in particular adapted to compensate for a bone missing, in particular bone loss.

The term "articular or joint implant" as used according to the present invention refers to an implant for treating an articular or joint bone defect. The articular or joint implant may be in the form of a permanent implant or temporary implant. Further, the articular or joint implant may be selected from the group consisting of tibial implant, femoral implant, knee joint implant, hip joint implant, ankle joint implant, shoulder joint implant, mandibular joint implant, elbow joint implant, finger joint implant, and spinal joint implant.

The term "bone defect" as used according to the present invention means any abnormality, in particular in the form of a loss of bone material and/or a void or cavity in a mammal, in particular human or non-human, bone, more particularly joint bone, preferably hip-joint or knee-joint bone. The bone defect may be the result of a bone fracture, a bone trauma, a bone disease, a tumor disease or a surgical intervention/reintervention, more particularly a revision operation, in particular after arthroplasty, in particular partial or total arthroplasty, such as hip or knee arthroplasty. In particular, the bone defect may be selected from the group consisting of tibial defect, femoral defect, knee joint defect, hip joint defect, ankle joint defect, shoulder joint defect, mandibular joint defect, elbow joint defect, finger joint defect and spinal bone defect.

Preferably, the biocompatible material being curable on contact with water or an aqueous liquid is a bone cement material.

The term "biocompatible material" as used according to the present invention refers to any material which is not or basically not harmful for a patient in need thereof.

The term "bone cement material" as used according to the present invention refers to any biocompatible material being curable on contact with water or an aqueous liquid, wherein the curing of the biocompatible material is based on a cement or cement-like setting process.

The term "aqueous liquid" as used according to the present invention refers to a liquid comprising or containing water. The aqueous liquid may be, for example, in the form of a body liquid, an aqueous solution or an aqueous suspension. Preferably, the aqueous liquid is an aqueous solution, in particular a salt solution such as sodium chloride solution. Alternatively, or in combination, the aqueous liquid may be a body fluid, in particular blood and/or tissue liquid, in particular hard tissue liquid, for example bone tissue liquid, and/or soft tissue liquid, for example cartilage liquid.

In sharp contrast to the augmentation component described in WO 2008/040408 A1, the augmentation component according to the present invention may be employed in combination with or in absence of the biocompatible material being curable on contact with water or an aqueous liquid.

Advantageously, through the first structure, the grip between the biocompatible material being curable on contact with water or an aqueous liquid and the articular or joint implant may be considerably improved. Thus, the risk of dislocation may be significantly reduced. An (additional) ingrowth of bone tissue is not necessary to reach sufficient stability. Preferably, there is no contact between the first structure of the augmentation component and the bone defect to be treated. Further, due to its permeable design for the biocompatible material being curable on contact with water or an aqueous liquid, the first structure advantageously contributes to an overall weight reduction of the augmentation component.

Advantageously, through the second structure, any aggressive contact between the first structure and the articular or joint implant, which could create material residues in the patient's body due to friction between the two implants, could be advantageously completely avoided.

Further, the second structure advantageously allows sufficient interlocking between the biocompatible material being curable on contact with water or an aqueous liquid and the first structure. Furthermore, any aggressive contact of the first structure with any soft body tissue such as cartilage tissue may be avoided or at least minimized.

In an embodiment of the invention, the first structure comprises or is in the form of a porous, in particular open porous, structure or lattice structure (grid structure), in particular flat or plate-shaped porous structure or flat or plate-shaped lattice structure.

Preferably, the first structure comprises voids or hollow spaces, in particular communicating voids or hollows spaces. In particular, the voids or hollow spaces may be in the form of pores, in particular interconnected pores.

The voids or hollow spaces may have a diameter of 0.25 mm to 5 mm. Further, the diameter of the voids or hollow spaces of the first structure may vary or may be constant throughout the first structure.

Further, the diameter of the voids or hollow spaces of the first structure may vary, in particular gradually vary, from one portion of the first structure to another portion of the first structure. Thus, it is advantageously possible to adjust the mechanical properties of the first structure. For example, a too high mechanical strength may be avoided, which would otherwise bear the risk of damaging neighboring bone tissue by osteolysis.

Further, the diameter of the voids or hollow spaces of the first structure may vary gradually or non-gradually, in particular rapidly or abruptly, at the transition of one portion of the first structure to another portion of the first structure.

Preferably, the first structure comprises or is constituted by struts, in particular interconnected struts. More preferably, the struts form voids or hollow spaces, in particular as disclosed in the preceding paragraphs. The struts of the first structure may also be termed as webs according to the present invention. The struts may have a thickness of 0.2 mm to 2.5 mm.

Especially preferably, the first structure is in the form of a lattice structure (grid structure), in particular three-dimensional lattice structure, comprising or being constituted by struts, preferably as disclosed in the preceding paragraphs.

Further, the first structure may have a relative density of 0.05 to 0.5.

The term "relative density" as used according to the present invention in the context of the first structure means the density of the first structure itself divided by the density of the material of which the struts are made.

In a further embodiment of the invention, the second structure has a height or thickness being greater than a height or thickness of the first structure. Preferably, the second structure is 0.1 mm to 20 mm, in particular 0.1 mm to 15 mm, preferably 0.1 mm to 12 mm, higher or thicker than the first structure. Further preferably, the first structure may have a height or thickness, in particular minimum height or thickness, of 0.5 mm to 4 mm, in particular 0.5 mm to 3 mm, preferably 1 mm to 2 mm.

In a further embodiment of the invention, the first structure is, in particular only, arranged at one side, in particular at a distal or proximal side, preferably at a distal side, of the augmentation component.

Alternatively, the first structure is arranged at two opposing sides, in particular at a distal side and a proximal side, of the augmentation component. For example, this may be the case, if the augmentation component has a thickness of 4 mm.

The term "proximal side" as used according to the present invention in the context of the augmentation component refers to a side of the augmentation component which in an implanted condition of the augmentation component faces towards the articular or joint implant or which faces towards a center of an articulation or of a joint to be treated by the augmentation component.

The term "distal side" as used according to the present invention in the context of the augmentation component refers to a side of the augmentation component which in an implanted condition of the augmentation component faces away from the articular or joint implant or which faces away from a center of an articulation or of a joint to be treated by the augmentation component.

The term "center of an articulation" or "center of a joint" as used according to the present invention refers to a junction surface or junction plane or junction line or junction point between articulating partners of an articulation or a joint.

In a further embodiment of the invention, one side, in particular only one side, in particular a proximal or distal side, preferably proximal side, of the augmentation component is free of the first structure.

In a further embodiment of the invention, the second structure extends in the direction of a proximal or distal side, preferably proximal side, of the augmentation component.

Further, the first structure may extend at least in sections, in particular only in sections or continuously, i.e. completely or without any interruptions, along a/the height or thickness of the second structure. In particular, the first structure may extend only in sections along a/the height or thickness of the second structure, wherein the proximal side and/or distal side, preferably both the proximal side and the distal side, of the augmentation component are/is free of the first structure. Alternatively, the first structure may extend continuously from one side to an opposing side, in particular from the distal side to the proximal side or vice versa, of the augmentation component.

In a further embodiment of the invention, the first structure forms a bottom element of the augmentation component. Preferably, the second structure comprises or forms a wall element, in particular a circumferential wall element, of the augmentation component. The wall element may have a thickness of 0.5 mm to 10 mm, in particular 0.5 mm to 5 mm, preferably 0.5 mm to 2 mm.

In a further embodiment of the invention, the second structure is not adapted to allow passage or penetration of the biocompatible material being curable on contact with water or an aqueous liquid and/or is not adapted to allow in-growth of bone tissue. Preferably, the second structure is free of any voids or hollow spaces, in particular in the form of pores. Thus, the advantages previously mentioned in the context of the second structure may be additionally improved.

In a further embodiment of the invention, the first structure and the second structure independently from each other comprise or consist of a material. Principally, the material may be selected from the group consisting of metallic material, ceramic material, polymeric material and combinations, in particular mixtures, of at least two of the aforesaid materials. Preferably, the material is a metallic material, in particular selected from the group consisting of titanium, titanium alloy, cobalt or cobalt-based alloy, implantable stainless steel (such as stainless steel according to ISO 5832-1) and combinations of at least two of the afore-said metallic materials. An especially preferred titanium alloy is Ti6Al4V, i.e. an alloy comprising the following: from 3.5 percent by weight to 4.5 percent by weight of vanadium, from 5.5 percent by weight to 6.75 percent by weight of aluminum and balance titanium and optionally unavoidable impurities. In particular, the Ti6Al4V may be Ti6Al4V according to ISO 5832-3. A useful ceramic material may be selected from the group consisting of biocompatible ceramics such as alumina, in particular according to ISO 6474-1, zirconia and combinations, in particular mixtures, of at least two of the aforesaid ceramic materials (useful combinations are known as ATZ or ZTA according to ISO 6474-2). A useful polymeric material may be selected from the group consisting of polyether ether ketone (PEEK), ultra-high-molecular-weight polyethylene (UHMWPE), vitamin E loaded UHMWPE, cross-linked UHMWPE, carbon loaded polymers and combinations, in particular mixtures, of at least two of the aforesaid polymeric materials.

In a further embodiment of the invention, the augmentation component further comprises a number of partition elements, i.e. (only) one partition element or a plurality of, i.e. two or more, partition elements, subdividing the augmentation component into a plurality of spaces or being involved in the formation of a plurality of spaces of the augmentation component. The number of partition elements advantageously increases or ensures compression strength of the augmentation component. Preferably, (also) the number of partition elements is not adapted to allow passage or penetration of the biocompatible material being curable on contact with water or an aqueous liquid and/or is not adapted to allow in-growth of bone tissue. More specifically, the number of partition elements is preferably free of any voids or hollow spaces, in particular in the form of pores. The number of partition elements may have a thickness of 0.5 mm to 10 mm, in particular 0.5 mm to 5 mm, preferably 0.5 mm to 2 mm.

Principally, the number of partition elements may comprise or consist of the same material as the first structure and/or second structure. Alternatively, the number of partition elements may comprise or consist of a different material than the first structure and/second structure. With respect to useful materials, reference is made in its entirety to the materials mentioned in terms of the first structure and second structure.

In a further embodiment of the invention, the augmentation component comprises at least one opening, in particular (only) one opening or several openings, being adapted to receive a securing element for securing the augmentation component to the articular or joint implant. Thus, connection between the augmentation component and the articular or joint implant may be advantageously improved. The securing element may be, for example, in the form of a screw, a nail or a pin. For example, the augmentation component may comprise two or three openings being adapted to receive a securing element for securing the augmentation component to the articular or joint implant.

In a further embodiment of the invention, the at least one opening being adapted to receive a securing element for securing the augmentation component to the articular or joint implant is in the form of at least one breakthrough, i.e. at least one opening completely traversing or spanning the augmentation component, in particular the first structure of the augmentation component, along its height or thickness direction, and/or is formed by a hollow cylindrical element, wherein the hollow cylindrical element is open on both ends. In particular, the hollow cylindrical element may have a wall which is not adapted to allow passage or penetration of the biocompatible material being curable on contact with water or an aqueous liquid and/or is not adapted to allow in-growth of bone tissue. Preferably, the wall of the hollow cylindrical element is free of any voids or hollow spaces, in particular in the form of pores. The wall may have a thickness of 0.5 mm to 10 mm, in particular 0.5 mm to 5 mm, preferably 0.5 mm to 2 mm. Principally, the hollow cylindrical element may comprise or consist of the same material as the first structure and/or second structure. Alternatively, the hollow cylindrical element may comprise or consist of a different material than the first structure and/or second structure. With respect to useful materials, reference is made in its entirety to the materials mentioned in terms of the first structure and second structure.

Alternatively, the augmentation component may be free of an opening being adapted to receive a securing element for securing the augmentation component to the articular or joint implant. In this case, securing of the augmentation component may be achieved in a different way, for example by means of a clip.

Preferably, the augmentation component has an outer contour section that partly matches an outer contour section of the articular or joint implant and has a further outer contour section that is adapted to fit a shaft, a keel, a fixation peg or a winglet of the articular or joint implant.

In a further embodiment of the invention, the augmentation component is in the form of a single-piece component.

In a further embodiment of the invention, the augmentation component is manufactured or produced by means of 3D printing or an additive production method, in particular 3D metal printing. This has the advantage that complex contours, designs, shapes and/or geometries for the augmentation component may be realized which would be unrealistic to be produced by subtractive manufacturing. Thus, 3D printing or an additive production method advantageously allows for a targeted manufacture of the augmentation component, thereby exactly addressing a patient's individual need. Additionally, by using 3D printing for the manufacture of the augmentation component, the applied material may be advantageously reduced by up to 50%. Consequently, this results in an up to 50% reduction in weight of the augmentation component. This in turn represents a significant progress both in terms of reduced amount and weight of the implant, thereby fostering the treatment of the articular or bone defect.

The term "3D printing" or "additive production method" as used according to the present invention refers to a method for the, in particular rapid, production of products, in particular finished products, tools, prototypes, models or samples based on the principle of material addition, by contrast to manufacturing processes such as milling or turning which are based on material removal. Production is carried out directly based on computer-internal data models using shapeless (liquids, powder and the like) or shape-neutral (bent or wire shaped) material by means of chemical and/or physical processes.

Further, the 3D printing or additive production method may be selected from the group consisting of powder bed methods, free space methods and liquid material methods.

The powder bed methods may be selected from the group consisting of selective laser melting, selective laser sintering, selective heat sintering, solidification of powder material by means of a binder (binder jetting) and electron beam melting.

The free space methods may be selected from the group consisting of fused deposition modeling, LOM (laminated object modeling) methods, cladding, wax deposition modelling, contour grafting, cold gas spraying and electron beam melting.

The liquid material methods may be selected from the group consisting of stereolithography, DLP (digital light processing) methods and LCM methods. The LCM methods may be a liquid composite molding method or a lithography-based ceramic manufacturing method.

Alternatively, the augmentation component may be produced by means of conventional manufacturing technique such as machining.

According to a second aspect, the invention relates to a surgical kit or system, in particular for treating an articular or joint bone defect.

The surgical kit or system comprises, in particular spatially separated from each other, an augmentation component according to the first aspect and at least one further kit or system component. In particular, the at least one further kit or system component is selected from the group consisting of articular or joint implant, a number of securing elements, i.e. one or a plurality of, i.e. two or more, securing elements, for securing the augmentation component to the articular or joint implant, a biocompatible material being curable on contact with water or an aqueous liquid, instructions for use and combinations of at least two of the aforesaid further kit or system components.

The number of securing elements may be selected from the group consisting of at least one screw, at least one nail, at least one pin, at least one clip and combinations of at least two of the aforesaid securing elements.

With respect to further features and advantages of the surgical kit or system, in particular in respect of the augmentation component, reference is made in its entirety to the respective embodiments described under the first aspect of the present invention, in order to avoid unnecessary repetitions. The features and advantages described under the first aspect of the invention, in particular in terms of the augmentation component, do also apply, mutatis mutandis, with respect to the surgical kit or system according to the second aspect of the present invention.

Further advantages and features of the invention can be found in the claims and the following description of preferred exemplary embodiments of the invention, which are presented with reference to the figures.

### Brief description of the figures

The figures schematically show the following:
- Fig. 1:: an embodiment of an augmentation component according to the invention,
- Fig. 2:: a perspective view of a proximal side of the augmentation component as shown in Fig. 1,
- Fig. 3:: an enlarged top view on a first structure of an augmentation component according to the invention,
- Fig. 4:: an enlarged detail view of an example of a first structure of an augmentation component according to the invention,
- Fig. 5:: an enlarged view of an augmentation component having a three-dimensional lattice structure and being applied on a biocompatible material being curable on contact with water or an aqueous liquid and
- Fig. 6:: an embodiment of a surgical kit according to the invention.

### More detailed description of the figures

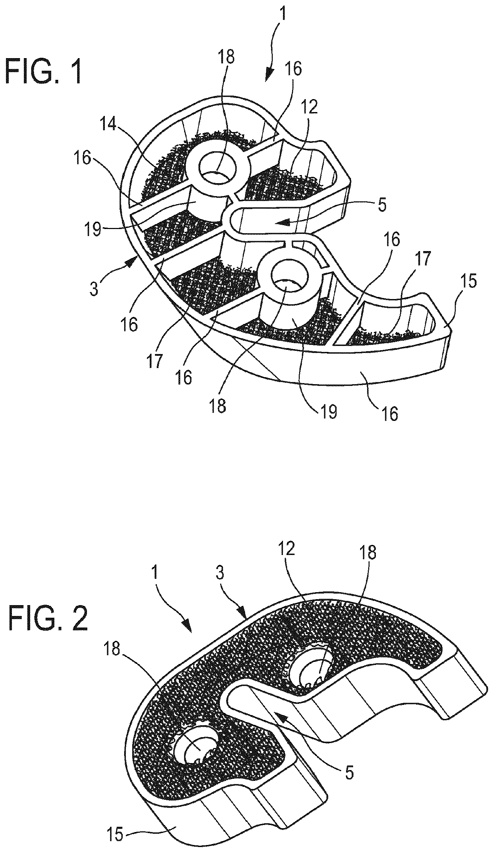

Fig. 1 schematically shows an augmentation component 1 for an articular or joint implant, in particular knee joint implant such as tibial implant, or hip joint implant.

The augmentation component 1 comprises
- a first structure 12 being adapted to allow passage or penetration of a biocompatible material being curable on contact with water or an aqueous liquid and/or being adapted to allow in-growth of bone tissue and
- a second structure 15 surrounding the first structure 12 at least in sections, in particular only in sections or completely, or being arranged at least in sections, in particular only in sections or completely, onto the first structure 12 along a periphery of the first structure.

Preferably, the augmentation component 1 has an outer contour section 3 that matches an outer contour section of the articular or joint implant and, preferably, the augmentation component 1 has a further outer contour section 5 that is adapted to fit a shaft, a keel, a fixation peg or a winglet of the articular or joint implant.

The first structure 12 is preferably in the form of a porous structure or lattice structure, in particular flat or plate-shaped porous structure or flat or plate-shaped lattice structure (grid structure). Thus, if the augmentation component 1 is applied, in particular compressed, on the biocompatible material being curable on contact with water or an aqueous liquid, unless it has not yet completely cured, the biocompatible material may pass or penetrate through the first structure 12. This optimizes connection between the augmentation component 1 and the bone ,and thus between the articular or joint implant and the bone.

Preferably, the first structure 12 has voids or hollow spaces 14, in particular communicating voids or hollow spaces 14.

In particular, the voids or hollow spaces 14 are in the form of pores, preferably interconnecting pores.

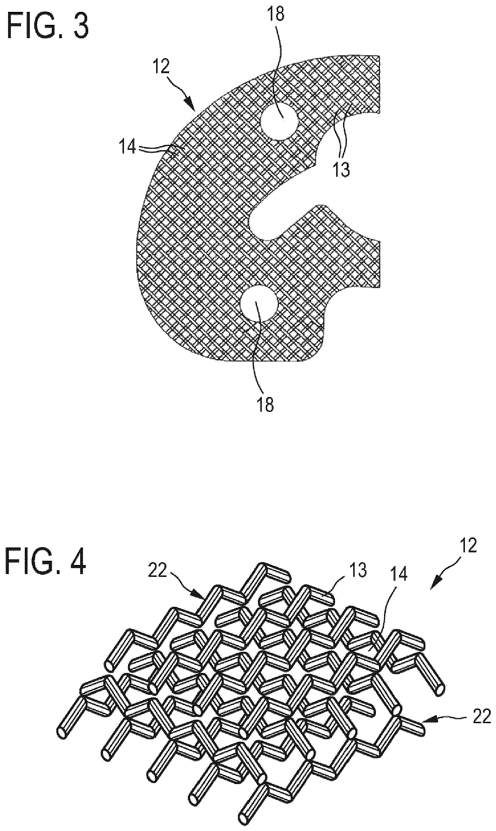

Further, the first structure 12 may comprise struts 13 or may be constructed by struts 13 (see Figs. 3 and 4). The struts 13 may be in particular in the form of interconnected struts. Preferably, the struts 13 form the voids or hollow spaces 14.

By the aid of the first structure, the grip between the biocompatible material being curable on contact with water or an aqueous liquid and the articular or joint bone implant may be advantageously improved. Thus, the risk of any instability may be properly addressed. An (additional) ingrowth of bone tissue is not mandatory in terms of reaching sufficient stability. Preferably, there is no contact between the first structure 12 of the augmentation component 1 and the bone defect to be treated. Further, due to its permeable design for the biocompatible material being curable on contact with water or an aqueous liquid, the first structure 12 advantageously contributes to an overall weight reduction of the augmentation component 1.

By the aid of the second structure 15, any aggressive contact with the articular or joint implant to be connected to the augmentation component 1 or bone defect to be treated can be avoided, which could otherwise be the cause for material residues in a patient's body, for example patient's knee, due to friction between the articular or joint implant and the augmentation component 1. A further advantage of the second structure 15 refers to a sufficient interlocking between the biocompatible material being curable on contact with water or an aqueous liquid and the first structure 12 of the augmentation component 1. Finally, also an aggressive contact between adjacent soft tissue such as cartilage tissue can be avoided by the second structure 15.

Preferably, as shown in Fig. 1, the second structure 15 has a height being greater than a height of the first structure 12.

Further preferably, the first structure 12 is arranged only at one side, in particular at a distal side, of the augmentation component 1, in particular wherein an opposing side, in particular a proximal side, of the augmentation component 1 is free of the first structure 12.

Further preferably, the second structure 15 extends in the direction of the proximal side of the augmentation component 1.

Further, the first structure 12 preferably forms a bottom element of the augmentation component 1 and the second structure 15 comprises or forms a wall element, in particular a circumferential wall element, of the augmentation component 1.

Preferably, the second structure 15 is not adapted to allow passage or penetration of the biocompatible material being curable on contact with water or an aqueous liquid and/or is not adapted to allow in-growth of bone tissue.

Further preferably, the first structure 12 and the second structure 15 independently from each other comprise or consist of a metallic material, in particular wherein the metallic material is selected from the group consisting of titanium, titanium alloy, cobalt or cobalt-based alloy, implantable stainless steel, in particular according to ISO 5832-1, and combinations of at least two of the aforesaid metallic materials.

Further preferably, the augmentation component 1 comprises a number of partition elements 16 subdividing the augmentation component 1 into a plurality of spaces 17 or being involved in the formation of a plurality of spaces 17 of the augmentation component 1.

The augmentation component 1 may - as shown in Fig. 1 - comprise two openings 18. Each of the openings 18 is preferably configured to receive a securing element, such as a screw, a nail or a pin, for securing the augmentation component 1 to the articular or joint implant. Preferably, each of the openings 18 is in the form of a breakthrough, i.e. an opening completely traversing or spanning the augmentation component 1, in particular the first structure 12, along its height or thickness direction. More preferably, each of the openings 18 is formed by a hollow cylindrical element 19. In particular, the hollow cylindrical elements 19 have a wall which is not adapted to allow passage or penetration of the biocompatible material being curable on contact with water or an aqueous liquid and/or is not adapted to allow in-growth of bone tissue.

Further preferably, the augmentation component 1 is in the form of a single-piece component.

Further preferably, the augmentation component 1 is manufactured by means of 3D printing, in particular 3D metal printing.

Fig. 2 shows a perspective view of a proximal side of the augmentation component 1 as shown in Fig. 1. With respect to further features and advantages, reference is therefore made in its entirety to the description of Fig. 1. In particular, the reference numbers in Fig. 2 have the same meaning as the respective reference numbers in Fig. 1.

Fig. 3 shows an enlarged top view on a first structure 12 of an augmentation component 1 according to the present invention. The first structure 12 is preferably made of a metallic material such as titanium or a titanium alloy, in particular Ti6Al4V, or a cobalt-based alloy.

The first structure 12 comprises connected struts 13 forming a three-dimensional lattice structure having communicating voids or hollow spaces, in particular interconnected pores, 14.

Further, the voids or hollow spaces 14 may have a diameter of 0.5 mm to 6 mm.

Further, the struts 13 may be of equal size, in particular length and/or thickness, and regularly arranged, in particular such that there is a uniform distribution of voids or hollow spaces 14 having an equal size throughout the volume of the first structure 12.

Alternatively, the struts 13 may be of different size, in particular length and/or thickness, and irregularly arranged, in particular such that the diameter of the voids or hollow spaces 14 may vary. Specifically, voids or hollow spaces 14 of different diameter may be uniformly distributed throughout the volume of the first structure 12.

Alternatively, the diameter of the voids or hollow spaces 14 may abruptly change from one portion of the augmentation component 1, in particular first structure 12, to another portion of the augmentation component 1, in particular first structure 12.

Fig. 4 shows an enlarged detail view of an example of a first structure 12 in the form of a three-dimensional lattice structure of an augmentation component 1 according to the present invention.

The lattice structure 12 comprises struts 13 being of equal length and regularly arranged. The struts 13 form individual traverses 22, in which the struts 13 being alternately inclined by 120° are connected to neighboring struts. Thus, within a traverse 22, the struts 13 run alternately upwards and downwards at an angle of 30°.

Within a plane, a plurality of such traverses 22 are arranged parallel to each other. Below this first plane, in a subjacent plane, traverses constructed in the same way run perpendicular to the traverses of the uppermost plane in such a way that the respective highest points meet the respective lowest points of the traverses in the plane above. In this area, the traverses running perpendicular to each other are connected to each other.

In the same way, there is a plane below, in which traverses are arranged perpendicular to the plane above, which are also connected in the same way to the traverses above.

The result is a three-dimensional lattice structure 12 which, viewed at 45° to the respective traverses, preferably has a honeycomb structure with continuous channels having a crosssection in the form of a regular hexagon. Advantageously, such a three-dimensional structure exhibits an especially high mechanical stability.

Preferably, the lattice structure 12 is manufactured by 3D metal printing, for example electron beam melting. This method uses a controlled electron beam, by which metal powder is melted in layers at the points where the lattice structure passes through the respective plane. Due to the layer-by-layer structure, even very complicated courses of the struts in the three-dimensional structure may be produced and, preferably, a molded body generated in this way may also already be adapted to the outer contour of an articular or joint implant.

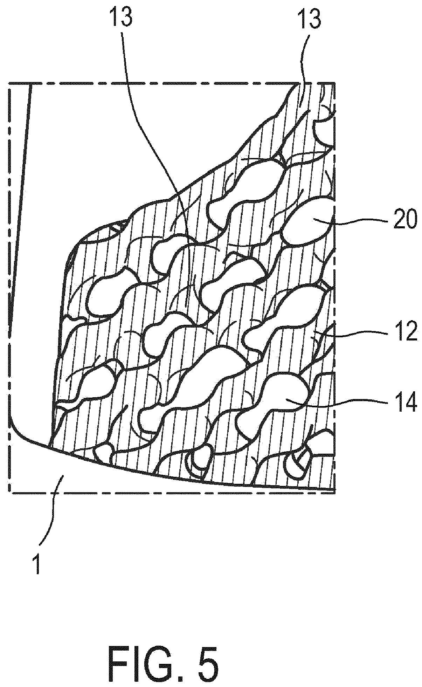

Fig. 5 shows an enlarged detail view of an augmentation component 1 having a first structure 12 in the form of a three-dimensional lattice structure being applied, in particular compressed, on a biocompatible material 20 being curable on contact with water or an aqueous liquid, in particular as long as the biocompatible material 20 has not yet completely cured. Preferably, the biocompatible material 20 is in the form of a bone cement. The biocompatible material 20, preferably during its curing, passes through or penetrates voids or hollow spaces, in particular pores, 14 of the lattice structure 12.

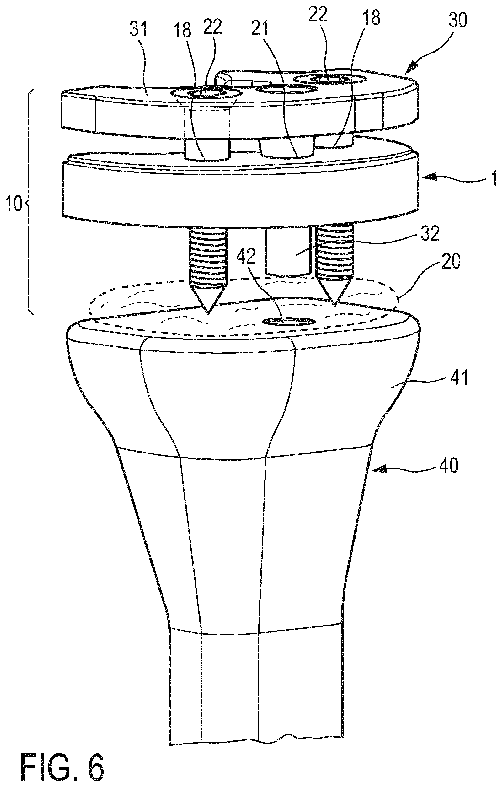

Fig. 6 shows an embodiment of a surgical kit 10 according to the present invention and its use for treating a tibial bone defect.

The surgical kit 10 comprises an augmentation component 1, a biocompatible material 20 being curable on contact with water or an aqueous liquid and a tibial implant 30.

The augmentation component 1 comprises two openings 18 each being configured to receive a securing element 22 for securing the augmentation component 1 to the tibia head 41.

Preferably, the augmentation component 1 has an outer contour section that partly matches an outer contour section of the tibial implant 30 and has a further outer contour section that is adapted to receive a shaft 32 of the tibial implant 30.

The tibial implant 30 comprises a plate-shaped tibial plateau 31 having the shaft 32 protruding vertically downwards. Preferably, the outer contour of the augmentation component 1 is adapted to the outer contour of the tibial plateau 31.

The tibial implant 30 is applied on the tibia head 41 of a respective resected tibia 40. With the tibial implant 30 fixed to the tibia 40, the shaft 32 engages in a complementarily formed recess 42 of the tibia 40, and thus positions the tibial implant 30.

For treating the tibial defect, the biocompatible material 20 may be applied at first on the tibia head 41. Next, curing of the biocompatible material 20 is initiated by adding water or an aqueous liquid. Alternatively, or in combination, curing of the biocompatible material 20 may be initiated by body liquids such as blood and/or body tissue liquid, in particular bone tissue liquid.

Then, the augmentation component 10 is applied, in particular compressed, onto the biocompatible material 20 as long as the biocompatible material 20 has not yet completely cured, in particular such that the biocompatible material 20 passes through voids or hollow spaces, in particular pores, of the augmentation component 1. This may be advantageously optimized by using securing elements 22 such as screws which engage in or through the openings 18 of the augmentation component 1 to (additionally) fix the augmentation component 1 to the tibia head 41.

Alternatively, for treating the tibial defect, the biocompatible material 20 may be mixed with water or an aqueous liquid, in particular aqueous solution, and subsequently applied on the tibia head 41 and/or on the tibial implant 30, wherein the tibial implant 30 may be subsequently pressed on the tibia head 41, in particular wherein the augmentation component 1 may be attached to the tibial implant 30.

Typically, a joint component being adapted to act as an artificial meniscus, for example a joint surface component, in particular made of ultra-high molecular weight polyethylene, is applied onto the tibial plateau 31. On the joint component, condyles of a femoral implant fixed to the femur may lean (not shown).

The augmentation component 1 is typically arranged at the underside of the tibial implant 30. Preferably, the augmentation component 1 is adapted to bridge the bone defect between the bottom of the tibial plateau 31 and the top side of the tibia head 41.

With respect to further features and advantages of the embodiments shown in the figures, reference is made in its entirety to the general description.

## Claims

1. Augmentation component (1) for an articular or joint implant (30), wherein the augmentation component (1) comprises
- a first structure (12) being adapted to allow passage or penetration of a biocompatible material (20) being curable on contact with water or an aqueous liquid and/or being adapted to allow in-growth of bone tissue and
- a second structure (15) surrounding the first structure (12) at least in sections or being arranged at least in sections onto the first structure (12) along a periphery of the first structure (12).

2. The augmentation component (1) according to claim 1, **characterized in that** the first structure (12) is in the form of a porous or lattice structure, in particular flat or plate-shaped porous or lattice structure.

3. The augmentation component (1) according to claim 1 or 2, **characterized in that** the second structure (15) has a height being greater than a height of the first structure (12).

4. The augmentation component (1) according to any of the preceding claims, **characterized in that** the first structure (12) is arranged only at one side, in particular at a distal side, of the augmentation component (1).

5. The augmentation component (1) according to any of the preceding claims, **characterized in that** one side, in particular a proximal side, of the augmentation component (1) is free of the first structure (12).

6. The augmentation component (1) according to any of the preceding claims, **characterized in that** the second structure (15) extends in the direction of a proximal side of the augmentation component (1).

7. The augmentation component (1) according to any of the preceding claims, **characterized in that** the first structure (12) forms a bottom element of the augmentation component (1) and the second structure (15) is in the form of a wall element, in particular a circumferential wall element, of the augmentation component (1).

8. The augmentation component (1) according to any of the preceding claims, **characterized in that** the second structure (12) is not adapted to allow passage or penetration of the biocompatible material (20) being curable on contact with water or an aqueous liquid and/or is not adapted to allow in-growth of bone tissue.

9. The augmentation component (1) according to any of the preceding claims, **characterized in that** the first structure (12) and the second structure (15) independently from each other comprise or consist of a metallic material, in particular wherein the metallic material is selected from the group consisting of titanium, titanium alloy, cobalt or cobalt-based alloy, implantable stainless steel and combinations of at least two of the afore-said metallic materials.

10. The augmentation component (1) according to any of the preceding claims, **characterized in that** the augmentation component (1) further comprises a number of partition elements (16) subdividing the augmentation component (1) into a plurality of spaces (17) or being involved in the formation of a plurality of spaces (17) of the augmentation component (1).

11. The augmentation component (1) according to any of the preceding claims, **characterized in that** the augmentation component (1) comprises at least one opening (18) being adapted to receive a securing element (22) for securing the augmentation component (1) to the articular or joint implant (30).

12. The augmentation component (1) according to claim 11, **characterized in that** the augmentation component (1) has an outer contour section (3) that partly matches an outer contour section of the articular or joint implant and has a further outer contour section (5) that is adapted to fit a shaft, a keel, a fixation peg or a winglet of the articular or joint implant.

13. The augmentation component (1) according to any of the preceding claims, **characterized in that** the augmentation component (1) is in the form of a single-piece component.

14. The augmentation component (1) according to any of the preceding claims, **characterized in that** the augmentation component (1) is manufactured by means of 3D printing, in particular 3D metal printing.

15. Surgical kit or system (10) comprising, in particular spatially separated from each other, an augmentation component (1) according to any of the preceding claims and at least one further kit or system component, in particular selected from the group consisting of articular or joint implant (30), a number of securing elements (22) for securing the augmentation component (1) to the articular or joint implant (30), a biocompatible material (20) being curable on contact with water or an aqueous liquid, instructions for use and combinations of at least two of the afore-said further kit or system components.
